(19) Europäisches Patentamt / European Patent Office / Office européen des brevets

(11) **EP 3 980 181 B1**

(12) **FASCICULE DE BREVET EUROPEEN**

(45) Date de publication et mention de la délivrance du brevet:
**27.03.2024 Bulletin 2024/13**

(21) Numéro de dépôt: **20730643.2**

(22) Date de dépôt: **05.06.2020**

(51) Classification Internationale des Brevets (IPC):
**B01L 3/00** *(2006.01)*      **C12M 1/00** *(2006.01)*
**G01N 1/40** *(2006.01)*      **G01N 15/00** *(2024.01)*

(52) Classification Coopérative des Brevets (CPC):
**B01L 3/502761; B01L 3/50273; C12M 47/04;**
**G01N 15/1404;** B01L 2200/0652; B01L 2300/163;
B01L 2400/0436; B01L 2400/0454;
G01N 2001/4094; G01N 2015/1006

(86) Numéro de dépôt international:
**PCT/EP2020/065732**

(87) Numéro de publication internationale:
**WO 2020/245432 (10.12.2020 Gazette 2020/50)**

(54) **PUCE MICROFLUIDIQUE POUR LA STRUCTURATION D'AGRÉGAT DE CELLULES PAR EXCLUSION OPTIQUE ET LÉVITATION ACOUSTIQUE**

MIKROFLUIDISCHER CHIP ZUR STRUKTURIERUNG VON ZELLAGGREGATEN DURCH OPTISCHE AUSSCHLUSS UND AKUSTISCHE LEVITATION

MICROFLUIDIC CHIP FOR STRUCTURING CELL AGGREGATES BY OPTICAL EXCLUSION AND ACOUSTIC LEVITATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.06.2019 FR 1906031**

(43) Date de publication de la demande:
**13.04.2022 Bulletin 2022/15**

(73) Titulaires:
• **Centre national de la recherche scientifique**
**75016 Paris (FR)**
• **Ecole Supérieure de Physique et de Chimie Industrielles de la Ville de Paris**
**75005 Paris (FR)**
• **SORBONNE UNIVERSITE**
**75006 Paris (FR)**
• **Université Paris Cité**
**75006 Paris (FR)**

(72) Inventeurs:
• **AIDER, Jean-Luc**
**92170 Vanves (FR)**
• **HOYOS, Mauricio**
**94000 Creteil (FR)**

• **JEGER-MADIOT, Nathan**
**75012 Paris (FR)**

(74) Mandataire: **IPAZ**
**Bâtiment Platon**
**Parc Les Algorithmes**
**91190 Saint-Aubin (FR)**

(56) Documents cités:
**EP-A1- 3 421 975      WO-A1-2017/006093**

• **STEFAN LAKÄMPER ET AL: "Direct 2D measurement of time-averaged forces and pressure amplitudes in acoustophoretic devices using optical trapping", LAB ON A CHIP, vol. 15, no. 1, 5 novembre 2014 (2014-11-05), pages 290-300, XP055292752, ISSN: 1473-0197, DOI: 10.1039/C4LC01144A**
• **Brodie Graham: "Hybrid optical and acoustic trapping", University of Dundee - DOCTOR OF PHILOSOPHY, 1 janvier 2014 (2014-01-01), XP055292865, Extrait de l'Internet: URL:http://discovery.dundee.ac.uk/portal/files/4180327/Brodie_PhD_2014.pdf [extrait le 2016-08-02]**

**Description**

La présente invention se rapporte à une puce microfluidique pour la manipulation d'objets tels que des micro ou nano particules de tailles comprises entre 0.1 μm et 300 μm. Elle concerne plus particulièrement la mise en lévitation de cellules par application d'ondes acoustiques. Par la suite il sera principalement question de cellule, mais la présente invention s'applique à toute particule sensible au moins aux ondes acoustiques.

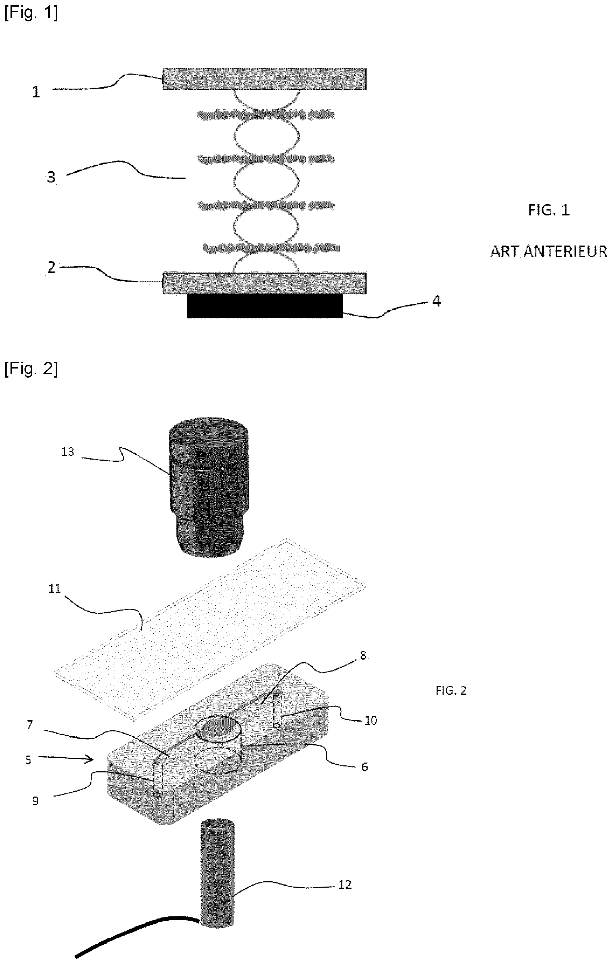

D'une façon générale ; le principe de la lévitation acoustique et de la formation d'agrégats de particules (ou cellules) en lévitation acoustique est rappelé sur la Fig. 1. En fonction de la fréquence, il est possible de créer 1 à N noeuds de pression entre les deux parois opposées 1 et 2 d'un canal 3. La condition à respecter est h = N.$\lambda_{ac}$/2, avec h la hauteur de la cavité et $\lambda_{ac}$ la longueur d'onde ultrasonore (onde acoustique). Un transducteur 4 est disposé à l'extérieur du canal en contact avec la paroi 2 de façon à générer des ondes acoustiques à travers cette paroi 2. La paroi 1 est un réflecteur renvoyant les ondes acoustiques dans le canal 3 de façon à générer une onde stationnaire et N noeuds de pression répartis sur la hauteur de la cavité. Ces variations de pression sont à l'origine de la création d'un champ de force de radiation acoustique. La composante axiale de la force de radiation acoustique ainsi créée va alors favoriser la formation d'agrégats dans chacun des noeuds de pression qui définissent des plans de lévitation acoustique. Les cellules sont réparties sur plusieurs agrégats, chaque agrégat est disposé sur un noeud de pression définissant un plan. Les traits réalisés sur la figure 1 sont simplement indicatifs de la force de radiation acoustique à l'origine des noeuds de pression.

Plus précisément, sous l'effet de la force de radiation acoustique, des particules (ou cellules) en suspension sont forcées à migrer vers les noeuds de pression acoustique. Une fois dans les différents plans de lévitation acoustique, la composante radiale de la force de radiation acoustique force les particules à se rassembler et à former un agrégat à chaque niveau. La fréquence de l'onde ultrasonore est définie de façon à obtenir plusieurs noeuds de pression distribués sur la hauteur h du canal.

Dans les applications acoustofluidiques actuelles, on utilise la force de radiation acoustique pour séparer les espèces en fonction de leurs propriétés physiques et mécaniques, à savoir leur densité et leur compressibilité, ainsi que de leurs tailles puisque la force acoustique dépend directement de ces paramètres comme défini dans l'équation suivante :

$$F_{ac} = 4\pi.r^3.k_{ac}.E_0.\Phi.\sin(2k_{ac}.z)$$

Avec r le rayon de la particule, $\Phi$ le facteur de contraste acoustique, $k_{ac}$ le nombre d'onde acoustique et $E_0$ l'énergie acoustique. Ceci définit la sensibilité des particules (ou cellules) à la force de radiation acoustique. Plus dense et plus grosse est la particule, plus grande est la force acoustique subie et plus vite la particule migre vers le noeud de pression. Cette force est habituellement utilisée pour séparer des particules ou cellules en écoulement en fonction de ces caractéristiques.

On connaît le document WO 2019/002551 A1 décrivant une méthode de séparation de particules en écoulement par application d'ondes acoustiques pour les placer en lévitation. Ce document décrit également le principe d'exclusion optique. La présente invention fait référence à ce document WO 2019/002551 A1 pour les modalités de mise en lévitation des particules et l'application du principe d'exclusion optique.

Lekämper et al. (Lab on a Chip, vol. 15(1), pages 290-300) décrit une cellule microfluidique avec une pince optique pour mesurer directement des forces d'une particule soumise à un champ acoustique. La pince optique utilise un laser permettant de manipuler les particules. Ces dernières sont disposées en différentes couches. La pince optique est appliquée entre deux lames de microscope. Les particules naviguent en amont et en aval du lieu d'application de la pince optique, dans un canal formé par les deux lames. Le lieu d'application de la pince optique et le conduit pour amener les particules forment un même canal continu.

La présente invention a pour but une nouvelle utilisation du principe de lévitation acoustique avec manipulation optique.

Un autre but de l'invention est un dispositif permettant une manipulation optimisée d'agrégat créé par lévitation acoustique.

L'invention a encore pour but une nouvelle méthode de création des agrégats.

On atteint au moins l'un des objectifs précités avec une puce microfluidique comprenant :

- un bloc en matériau biocompatible,
- un canal de passage réalisé dans le bloc pour le passage de cellules baignant dans un liquide, notamment nutritif (milieu de culture),
- une cavité résonante par exemple destinée à la culture cellulaire, réalisée dans le bloc, reliée au canal de passage et comprenant des parois pour contenir les cellules provenant du canal de passage,

- un générateur d'ondes acoustiques apte à former au moins un agrégat de cellules en lévitation acoustique dans la cavité résonante,
- au moins un émetteur optique apte à illuminer des cellules dans la cavité résonante à travers au moins une paroi de la cavité résonante et simultanément à la génération d'ondes acoustiques de façon à structurer ledit au moins un agrégat par la technique d'exclusion optique.

La cavité résonante comprend des parois pour contenir les cellules. En d'autres termes, les cellules peuvent pénétrer une zone dans la cavité résonante où elles ne sont plus sous influence de l'écoulement dans le canal de passage.

Elles peuvent donc être soumises aux actions des ondes acoustiques et optiques.

Elles peuvent également rester immobiles, relativement au mouvement de l'écoulement dans le canal de passage. Immobile signifie qu'elles sont soumises aux faibles mouvements du milieu, mais pas au mouvement de déplacement linéaire de l'écoulement.

On peut ajuster le débit de l'écoulement pour que celui-ci n'entraîne pas les cellules formant notamment un ou plusieurs agrégats. La force acoustique est alors suffisamment importante pour s'opposer à l'écoulement.

Avec la puce microfluidique selon l'invention, il est possible d'utiliser simultanément les ondes acoustiques et les faisceaux optiques de façon à maintenir les cellules en lévitation sur une ou plusieurs couches par ondes acoustiques tout en les manipulant par faisceaux optiques selon la technique d'exclusion optique.

La puce microfluidique selon l'invention constitue un bioréacteur opto-acoustique dans lequel les cellules peuvent être mises en culture en lévitation acoustique et manipulées par illumination spécifique de façon à structurer les agrégats en couches successives de cellules. Par structuration, on entend une répartition spatiale des couches de cellules différentes les unes par rapport aux autres.

Dans la cavité résonante, les cellules baignent dans un liquide ne perturbant pas les ondes acoustiques ni les faisceaux optiques utilisés et adapté à la culture cellulaire. La cavité résonante est avantageusement destinée à une culture de cellules en lévitation acoustique. Il est clair pour l'homme du métier que toute micro ou nano particule sensible aux ondes acoustiques et éventuellement à certains faisceaux optiques, de façon endogène ou exogène, peut être utilisée dans l'invention.

En utilisant à la fois les propriétés optiques et acoustiques, il est donc possible de structurer spatialement des agrégats de cellules en lévitation acoustique. Cette opération peut être répétée plusieurs fois, par injections successives de différents types de cellules marquées (marqueurs biologiques fluorescents) ou non et réagissant à différentes longueurs d'onde optiques.

La force de radiation acoustique due aux ondes acoustiques permet de créer des agrégats de particules que l'on peut maintenir en lévitation acoustique aussi longtemps que nécessaire. Cette force est dimensionnée pour maintenir cet agrégat en lévitation acoustique dans la cavité résonante qui constitue un piège acoustique ( « acoustic trap » en anglais).

Selon une caractéristique avantageuse de l'invention, la puce microfluidique peut en outre comprendre au moins un deuxième émetteur optique, les deux émetteurs étant disposés de façon à émettre à travers respectivement deux parois opposées de la cavité résonante.

De préférence, les deux émetteurs optiques émettent à des longueurs d'ondes différentes. Une telle disposition permet d'agir par la technique d'exclusion optique sur différents types de cellules de façon simultanée ou séquentielle. Cela permet également d'agir sur des agrégats de façon symétrique car il y a atténuation de l'intensité des faisceaux traversant une épaisseur de fluide et rencontrant des agrégats le long de son axe de propagation. Grâce à ce montage on agit de la même façon sur l'agrégat du haut que sur celui du bas.

Selon un mode de réalisation de l'invention, le générateur d'ondes acoustiques peut comprendre un élément supérieur et un élément inférieur prenant en sandwich au moins une partie de la cavité résonante sur deux parois opposées ; l'élément supérieur, fixe ou amovible, étant un transducteur supérieur ou un réflecteur d'ondes acoustiques ; et l'élément inférieur étant un transducteur inférieur, les transducteurs supérieur et inférieur étant aptes à émettre les ondes acoustiques. Par ailleurs, l'élément supérieur et/ou l'élément inférieur sont transparents aux faisceaux lumineux prévus pour l'illumination des cellules.

En d'autres termes, le générateur comprend un transducteur inférieur associé à un réflecteur apte à réfléchir les ondes acoustiques du transducteur inférieur de façon à créer la force de radiation acoustique dans la cavité résonante. Mais le réflecteur peut être remplacé par un transducteur supérieur générant les mêmes ondes acoustiques que le transducteur inférieur de manière synchrone de façon à créer également la force de radiation acoustique dans la cavité résonante.

De préférence, au moins l'un des deux transducteurs est transparent à la longueur d'onde optique ou bien est annulaire pour laisser passer en son centre l'illumination optique.

Le caractère amovible de l'élément supérieur permet éventuellement d'évacuer ou de récupérer les cellules formant agrégat.

Selon un mode de réalisation de l'invention, la cavité résonante est fermée sur son extrémité inférieure par le transducteur inférieur. Le transducteur inférieur constitue ainsi la paroi inférieure de la cavité résonante. Dans une telle réalisation, le transducteur inférieur est en contact direct avec le milieu de culture. Cette configuration permet d'améliorer le rendement énergétique de la cavité résonante.

Selon une caractéristique avantageuse de l'invention, le transducteur inférieur peut être disposé à l'intérieur de la cavité résonante.

On prévoit ainsi un transducteur inférieur amovible capable de glisser à l'intérieur de la cavité résonante. De préférence, les dimensions du transducteur permettent d'assurer l'étanchéité de la cavité résonante. Dans ce cas, le volume utile formant le milieu de culture est inférieur au volume total de la cavité résonante. Il est ainsi possible de définir différents volumes de la cavité résonante. Il s'agit alors d'une cavité à volume variable et donc nombre de plan de lévitation variable.

Avantageusement, la cavité résonante peut comporter au moins une butée pour bloquer la tête du transducteur inférieur une fois insérée dans la cavité résonante.

Selon un mode de réalisation de l'invention, la cavité résonante peut être fermée sur son extrémité inférieure par un film fixe ou amovible transparent aux ondes acoustiques provenant du transducteur inférieur disposé à l'extérieur de la cavité de résonnance.

Ce film peut être en matériau polydiméthylsiloxane (PDMS) ou en copolymère oléfinique cyclique (COC) ou être un film adhésif PCR (« Polymerase Chain Reaction » en anglais) facilement décollable et permettant un très bon transfert des ondes ultrasonores.

Un film amovible permet le passage ou la récupération d'agrégat de cellules en fin de culture par exemple.

De préférence, la cavité résonante est un cylindre dont les parois latérales sont constituées par le bloc. La base supérieure peut être formée par le réflecteur ou le transducteur supérieur. La base inférieure peut être formée par un film ou directement par le transducteur inférieur.

Selon un mode de réalisation, le canal de passage peut déboucher dans la cavité résonante à l'extrémité supérieure d'une paroi latérale du cylindre. Un tel arrangement permet d'alimenter la cavité en cellules par l'extrémité supérieure de la paroi latérale du cylindre.

Selon l'invention, la cavité résonante peut comporter une butée disposée de façon à ce que la tête du transducteur inférieur puisse être insérée jusqu'à réduire la hauteur du volume utile dans la cavité résonante égale à la hauteur du canal de passage.

Idéalement, le volume utile permet la réalisation d'un ou plusieurs agrégats de cellules en monocouche ou multi-couches.

Selon un mode de réalisation avantageux de l'invention, le canal de passage peut être réalisé sur la surface supérieure du bloc et une lame collée ou amovible peut couvrir l'ensemble de la surface du bloc y compris l'extrémité supérieure de la cavité résonante ; cette lame étant transparente aux faisceaux optiques prévus pour illuminer depuis l'extérieur les cellules de la cavité résonante. En complément, lorsqu'un transducteur est disposé en face, cette lame est réfléchissante sur le côté interne de la cavité résonante aux ondes acoustiques du transducteur.

De préférence, le canal de passage et le cylindre peuvent être disposés perpendiculairement l'un par rapport à l'autre. Le bloc peut alors comprendre en outre deux microcanaux traversant le bloc de part en part, parallèlement au cylindre et connectés respectivement aux deux extrémités libres du canal de passage ; le premier microcanal étant destiné à l'arrivée de cellules dans le canal de passage et le deuxième microcanal étant destiné à l'évacuation de cellules depuis le canal de passage.

A titre d'exemple non limitatif, le réflecteur est une lame en verre, en Polyméthacrylate de méthyle (PMMA), en quartz, en silicium, en polydiméthylsiloxane (PDMS) ou en copolymère oléfinique cyclique (COC). Une telle lame est conçue pour assurer une bonne transmission d'un côté et une bonne réflexion de l'autre. De préférence, le réflecteur peut être conçu à partir d'un matériau identique à celui du bloc et présentant une surface interne traitée pour la réflexion d'ondes acoustiques.

La puce selon l'invention peut comprendre plusieurs microcanaux réalisés dans l'épaisseur du bloc et débouchant dans la cavité résonante pour des entrées et/ou des sorties de cellules. De préférence, ces microcanaux sont alignés respectivement avec les noeuds de pression prévus dans la cavité résonante du fait de la force de radiation acoustique. Ces microcanaux peuvent par exemple servir pour l'injection de cellules de natures différentes de celles injectées par le canal de passage, pour l'injection de nutriments,...et également pour l'évacuation de cellules.

Avantageusement, la hauteur de la cavité résonante peut être fonction du nombre de noeuds de pression à créer et de la longueur d'onde des ondes acoustiques générées par le générateur. La cavité résonante est conçue pour contenir une superposition de plusieurs couches cellulaires, alors que la hauteur du canal de passage est de préférence adaptée à la taille d'une cellule.

La cavité résonante est plus haute que le canal de passage, par exemple plusieurs diamètres d'une cellule, notamment 10 diamètres minimum. On peut chercher à mettre le premier noeud de pression au niveau du canal de passage, donc une hauteur de canal de l'ordre de $\lambda/4$.

De préférence, le canal de passage peut être de forme rectiligne de sorte que la section du canal de passage entre une extrémité d'entrée et la cavité résonante est colinéaire à la section du canal de passage entre la cavité résonante et l'autre extrémité de sortie du canal de passage.

Selon un mode de réalisation de l'invention, le bloc peut être en polydiméthylsiloxane (PDMS) ou en copolymère oléfinique cyclique (COC).

En particulier, le bloc est conçu à partir d'un matériau perméable aux gaz afin de faciliter les échanges gazeux entre le milieu, le contenu de la cavité résonante, et l'extérieur si nécessaire. Les canaux sont gravés ou moulés dans le bloc et peuvent être installés dans un incubateur de façon à assurer les conditions de culture optimale en termes de gaz et température.

Notamment, la cavité résonante peut être dimensionnée avec une hauteur supérieure au diamètre du canal de passage débouchant dans cette cavité résonante. On distingue ainsi clairement le canal de passage qui est un conduit de faible diamètre, typiquement adapté pour véhiculer les cellules uniquement en ligne, et la cavité résonante qui est de dimension plus grande apte à contenir des agrégats de cellules dans un plan et dans un volume.

A titre d'exemple non limitatif, la cavité résonante peut présenter un diamètre entre 1 et 50mm, une hauteur de la cavité résonante comprise entre 5 et 15mm et une hauteur du canal de passage égale à $450\mu m$.

La puce microfluidique selon l'invention peut en outre comprendre au moins un microcanal supplémentaire réalisé dans le bloc sur le même plan que le canal de passage. Il s'agit ici d'un arrangement simplifié permettant d'alimenter et évacuer la cavité résonante de cellules pouvant s'agréger sur un même plan que les cellules provenant du canal de passage qui est en fait un microcanal principal.

Ce microcanal supplémentaire peut également servir à injecter d'autres cellules, des biomarqueurs, ou encore de laver le milieu de culture ou récupérer la production des cellules au cours de leur culture.

Selon un autre aspect de l'invention, il est proposé un procédé de manipulation de cellules en lévitation acoustique dans une puce microfluidique telle que définie cidessus. Ce procédé comprend les étapes suivantes :

- injection de cellules dans la cavité résonante via une entrée du canal de passage,
- génération d'ondes acoustiques pour mettre en lévitation acoustique les cellules injectées de façon à former un agrégat de cellules en au moins une couche,
- au moins une phase d'illumination à une longueur d'onde optique spécifique des cellules simultanément au maintien des ondes acoustiques de façon à manipuler des cellules selon le principe d'exclusion optique.

Selon l'invention, on utilise le principe d'exclusion opto-acoustofluidique sélectif décrivant le fait qu'une particule ou cellule réagit à certaines longueurs d'onde optique tandis que d'autres particules ou cellules ne réagissent pas du tout.

Selon un mode de mise en oeuvre avantageux de l'invention, les cellules injectées sont de natures différentes, et l'étape de génération d'ondes acoustiques et d'illumination peuvent être réalisées de la manière suivante :

- génération d'ondes acoustiques pour mettre en lévitation acoustique des cellules injectées et en même temps application d'un faisceau lumineux à une longueur d'onde spécifique permettant le principe d'exclusion des cellules sensibles à cette longueur d'onde de sorte que seules les cellules *insensibles* à cette longueur d'onde optique forment un agrégat en une couche,
- maintien des ondes acoustiques et arrêt du faisceau lumineux de sorte que les cellules sensibles à la longueur d'onde du faisceau lumineux viennent maintenant s'agréger en périphérie de l'agrégat déjà formé, et obtenir ainsi un agrégat structuré radialement.

La présente invention permet ainsi la réalisation d'agrégats de cellules monocouche structurés radialement. L'agrégat peut ainsi être composé d'anneaux successifs de cellules différentes et ainsi permettre la coculture de cellules, étapes indispensables à la formation d'organoïdes.

Par agrégat de cellules on entend une couche de cellules satisfaisant à toutes les caractéristiques suivantes: au moins deux cellules comprises dans ladite couche, en particulier au moins 10%, mieux 25%, de préférence 50% des cellules comprises dans ladite couche, sont en contact, et ladite couche présente, sur au moins une partie de sa longueur, une succession de cellules lors du déplacement selon au moins l'une de ses dimensions transversales.

Selon un mode de mise en oeuvre avantageux de l'invention, on réalise une structure tridimensionnelle formée de plusieurs couches d'agrégats en réalisant les étapes suivantes :

- l'étape d'injection comprend l'injection de cellules dans la cavité résonante via une ou plusieurs entrées, et
- l'étape de génération d'ondes acoustiques comprend en outre la génération d'ondes acoustiques pour mettre en lévitation acoustique plusieurs agrégats de cellules injectées sur plusieurs niveaux, les niveaux étant des noeuds de pression acoustique dont le nombre est fonction de la longueur d'onde des ondes acoustiques et de la hauteur de la cavité résonante.

EP 3 980 181 B1

L'invention permet la création de plusieurs monocouches (feuillets cellulaire / cell sheets) les unes au-dessus des autres, ce qui procure un gain de temps considérable pour la culture cellulaire puisqu'il n'est plus nécessaire de trypsiniser une feuille de cellules, la décoller d'un éventuel substrat solide pour ensuite la déposer sur un autre. Ce processus est plus particulièrement adapté aux cultures de cellules souches de type MSC (« Mesenchymal Stromal Cells » en anglais) où l'on cultive les cellules pour fabriquer des feuillets de cellules qui sont ensuite superposées, mais est pertinent pour tout type de culture cellulaire destinée à former des feuillets de cellules.

Avec l'invention, il est possible d'envisager la fabrication en lévitation acoustique de sphéroïdes, d'organoïdes ou de tumoroïdes qui pourront être maintenus et cultivés en lévitation acoustique.

L'invention permet la réalisation d'une culture cellulaire en maintenant l'agrégat ou les agrégats obtenus immobiles en lévitation acoustique pendant la durée de la culture.

Un tel système a ainsi permis la culture en lévitation acoustique de MSC pendant 18 jours. A l'issue de cette culture, les cellules étaient vivantes et ont pu proliférer normalement une fois déposées sur un substrat. Le système a ainsi été validé pour des cultures sur des temps courts (quelques heures) et des temps longs (quelques semaines). Ceci est rendu possible grâce au flux de nutriments et à la porosité des matériaux utilisés qui permet les échanges gazeux dans un incubateur. Le bioréacteur acoustique a été placé dans un incubateur pour permettre la culture des cellules. La compacité du présent système permet de placer plusieurs puces dans un incubateur. On peut ainsi réaliser la culture de nombreux feuillets ou de sphéroïdes en lévitation acoustique en parallèle (quelques dizaines par puits, et quelques puits dans l'incubateur).

D'autres caractéristiques et avantages de l'invention apparaîtront à la lecture de la description détaillée de mises en oeuvre et de modes de réalisation nullement limitatifs, au regard de figures annexées sur lesquelles :

[FIG.1] La figure 1 est une vue schématique de création de noeuds de pression dans un canal par lévitation acoustique selon l'art antérieur ;

[FIG.2] La figure 2 est une vue schématique éclatée d'un exemple du dispositif selon l'invention,

[FIG.3] La figure 3 est une vue de dessus d'un exemple du dispositif selon l'invention,

[FIG.4] La figure 4 est une vue schématique en coupe selon l'axe longitudinal du canal de passage, avec un émetteur optique et un transducteur d'ondes acoustiques disposé à l'extérieur de la cavité résonante, selon l'invention,

[FIG.5] La figure 5 est une vue schématique en coupe selon l'axe longitudinal du canal de passage, avec un émetteur optique et un transducteur d'ondes acoustiques fermant la cavité résonante sur sa base, selon l'invention,

[FIG.6] La figure 6 est une vue schématique en coupe selon l'axe longitudinal du canal de passage, avec un émetteur optique et un transducteur d'ondes acoustiques disposé à l'intérieur de la cavité résonante, selon l'invention,

[FIG.7] La figure 7 est une vue schématique en coupe selon l'axe longitudinal du canal de passage, avec un émetteur optique et un transducteur d'ondes acoustiques disposé à l'intérieur de la cavité résonante, en buté supérieur, selon l'invention,

[FIG.8] La figure 8 est une vue schématique en coupe selon l'axe longitudinal du canal de passage, avec un émetteur optique et un transducteur d'ondes acoustiques fermant la cavité résonante sur sa base, le canal de passage étant disposé dans l'épaisseur du bloc, selon l'invention,

[FIG.9] La figure 9 est une vue du dispositif de la figure 8 avec deux émetteurs optiques et un transducteur d'ondes acoustiques, selon l'invention,

[FIG.10] La figure 10 est une vue schématique d'un transducteur conçu à partir d'un matériau transparent pour le faisceau optique prévu pour la mise en oeuvre du principe d'exclusion, selon l'invention,

[FIG.11] La figure 11 est une vue schématique d'un transducteur ayant la forme d'un anneau, selon l'invention,

[FIG.12] La figure 12 est une vue schématique en coupe selon l'axe longitudinal du canal de passage, avec deux émetteurs optiques et un transducteur de la forme d'un cylindre creux disposé autour de la cavité résonante, selon l'invention,

[FIG.13] La figure 13 est une vue schématique en coupe d'un bloc compatible avec les dispositifs des figures 2 à 12 avec une pluralité de microcanaux gravés dans le bloc et débouchant dans la cavité résonante, selon l'invention,

[FIG.14] La figure 14 est une vue schématique en coupe d'un agrégat de cellules structuré radialement, selon l'invention, et

[FIG.15] La figure 15 est une vue schématique en coupe de plusieurs agrégats de cellules structurés radialement et latéralement, selon l'invention.

[FIG.16] La figure 16 est une courbe illustrant des vitesses d'éjection de deux types de cellules en fonction de la longueur d'onde ;

[FIG.17] La figure 17 est une courbe illustrant des vitesses d'éjection de plusieurs différents types de cellules avec des tailles différentes en fonction de la longueur d'onde ;

[FIG.18] La figure 18 est une courbe illustrant la vitesse d'éjection des globules rouges en fonction de la longueur d'onde ;

[FIG.19] La figure 19 est une courbe illustrant la vitesse d'éjection des globules rouges en fonction de la longueur d'onde ;

[FIG.20] La figure 20 est une courbe illustrant la vitesse d'éjection des globules blancs en fonction de la longueur d'onde ;

[FIG.21] La figure 21 comprend deux photos illustrant la formation d'un agrégat de particules blanches à partir d'un mélange de particules de polystyrènes blanches de $10\mu m$ avec des particules rouges de $3\mu m$ ;

[FIG.22] La figure 22 comprend quatre photos prises à des instants différents lors de la formation d'un agrégat « purifié » en lévitation acoustique par exclusion optique de globules rouges ;

[FIG.23] La figure 23 comprend trois photos illustrant l'effet d'exclusion optique appliqué à une cavité multi-noeuds ;

[FIG.24] La figure 24 illustre une structure annulaire stratifiée comprenant des particules de polystyrènes rouges de $15\mu m$ de diamètre et des particules de polystyrènes blanches de $40\mu m$.

Les modes de réalisation qui seront décrits dans la suite ne sont nullement limitatifs; on pourra notamment mettre en oeuvre des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieure.

En particulier toutes les variantes et tous les modes de réalisation décrits sont prévus pour être combinés entre eux dans toutes les combinaisons où rien ne s'y oppose sur le plan technique.

Sur les figures, les éléments communs à plusieurs figures conservent la même référence.

Bien que l'invention n'y soit pas limitée, on va maintenant décrire une puce microfluidique adaptée pour la culture d'agrégats de cellules en lévitation acoustique. Sur la figure 2, sont représentés un ensemble de composants d'un exemple d'une puce microfluidique selon l'invention. On distingue un bloc 5 par exemple en matériau PDMS et de forme parallélépipédique avec une surface supérieure et une surface inférieure. Une cavité résonante 6 de forme cylindrique a été réalisée au centre de ce bloc sur toute l'épaisseur du bloc 5 entre les deux surfaces. Cette cavité résonante 6 a pour objet de piéger des cellules par la force acoustique.

Pour alimenter la cavité résonante 6, un canal de passage 7, 8 est gravé sur la surface supérieure du bloc 5 de sorte que ce canal de passage et l'intérieur de la cavité sont accessibles. Le canal de passage comporte une première partie 7 destinée à l'entrée des cellules depuis un microcanal d'entrée 9 vers la cavité résonante. Il comporte également une seconde partie 8 destinée à l'évacuation de cellules depuis la cavité résonante 6 vers un microcanal de sortie 10. Les deux microcanaux d'entrée et de sortie sont réalisés dans l'épaisseur du bloc, comme la cavité résonante et débouchent sur la surface inférieure du bloc 6, l'arrière de ce bloc étant plus accessible à différents dispositifs d'alimentation et de gestion de la puce microfluidique selon l'invention.

De préférence, ce bloc est conçu à partir d'un matériau bio-compatible apte à assurer des échanges gazeux si nécessaire entre la cavité résonante et l'extérieur (incubateur). Il est conçu pour que la puce microfluidique selon l'invention puisse assurer un flux de nutriments et un flux de milieu de culture si nécessaire au sein de la cavité résonante. Il permet bien évidemment l'injection des cellules et leur évacuation et permet la création des agrégats de grande dimension au sein de la cavité résonante.

La puce microfluidique est installée dans un incubateur de façon à assurer les conditions de culture optimale (gaz et température).

On distingue également une lame de verre destiné à couvrir, notamment par collage au plasma, la surface supérieure du bloc 5. Il peut s'agir d'une lame réalisée ensemble avec le bloc 5. Cette lame de verre 11 présente, au moins sur sa paroi interne faisant face à la cavité résonante, une surface interne apte à réfléchir des ondes acoustiques d'un transducteur 12 prévu en face, côté de la surface inférieure du bloc 6. Avantageusement, la lame de verre est transparente aux faisceaux optiques d'un émetteur optique 13 disposé au-dessus de la lame 11.

La lame 11 peut être conçu à partir d'un ou une combinaison de matériaux suivants : verre, PMMA, quartz, silicium, COC, PDMS, de façon à assurer une bonne transmission d'un côté et une bonne réflexion de l'autre.

Le transducteur 12 est composé d'un cylindre en inox contenant un élément piézoélectrique dont la fréquence de fonctionnement peut être choisie en fonction de la hauteur de la cavité résonante. Cette fréquence peut être choisie entre 0.1 MHz à 10 MHz pour des cavités de résonance dont l'épaisseur (la hauteur) peut varier de quelques mm à quelques dizaines de $\mu m$.

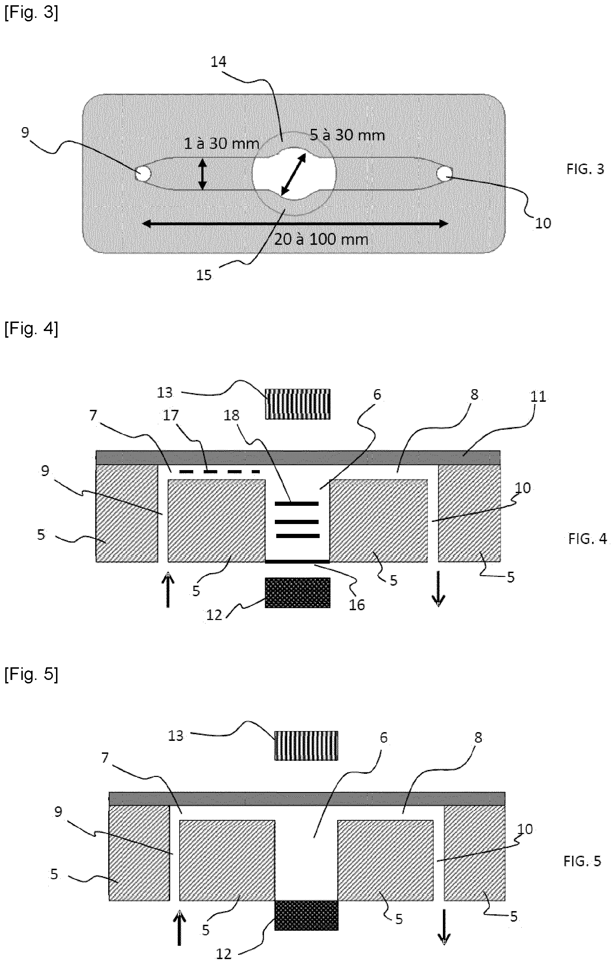

La figure 3 est une vue de dessus du bloc 5. On distingue les extrémités des microcanaux 9 et 10 à chaque bout du canal de passage 7, 8. Ce canal de passage présente une forme évasée depuis les microcanaux vers la cavité résonante de façon à assurer une bonne progression des cellules à l'entrée et à l'évacuation. Le canal de passage peut présenter une section transversale (circulaire, rectangulaire, carrée ou autre) d'une hauteur de 1 à 30mm par exemple. Des butées 14 et 15 comme on le verra plus tard sont disposées sur la paroi interne de la cavité résonante et permettent de définir un diamètre au niveau supérieur de la cavité résonante d'environ 5 à 30 mm.

D'autres entrées/sorties (non représentées) de type microcanaux 9, 10 et canal de passage 7,8 peuvent être réalisées pour alimenter la cavité résonante par des cellules identiques ou différentes, des biomarqueurs, ou encore pour laver le milieu de culture ou récupérer la production des cellules au cours de leur culture.

Sur la figure 4, est illustré un mode de réalisation dans lequel la base inférieure de la cavité résonante est fermée par un film 16 étanche. Une telle configuration permet d'éviter toute contamination du milieu de culture par le transducteur 12 qui se trouve à l'extérieur de la cavité. Ce film 16 peut être amovible de façon à permettre l'évacuation d'agrégats formés dans la cavité résonante. Idéalement ce film est transparent pour les ondes acoustiques prévues pour la création de force de radiation acoustique dans la cavité. Il peut être en PDMS, en COC ou un film adhésif PCR. Sur la figure 4, est illustré l'écoulement 17 de cellules et la création d'agrégat 18 à partir de cellules piégées dans la cavité résonante 6 sous l'action des ondes acoustiques émises par le transducteur 12.

Dans un mode de réalisation comme on le voit sur la figure 5 par exemple, le transducteur 12 packagé peut être en contact direct avec le milieu de culture dans la cavité résonante. Dans ce cas, il n'y a pas de paroi de transmission, seule la lame de verre 11, réflectrice, est placée en face du transducteur 12. Cette configuration permet d'améliorer le rendement énergétique de la cavité résonante en supprimant une couche de transmission, par exemple le film 16 de la figure 4, dans laquelle l'énergie peut se dissiper.

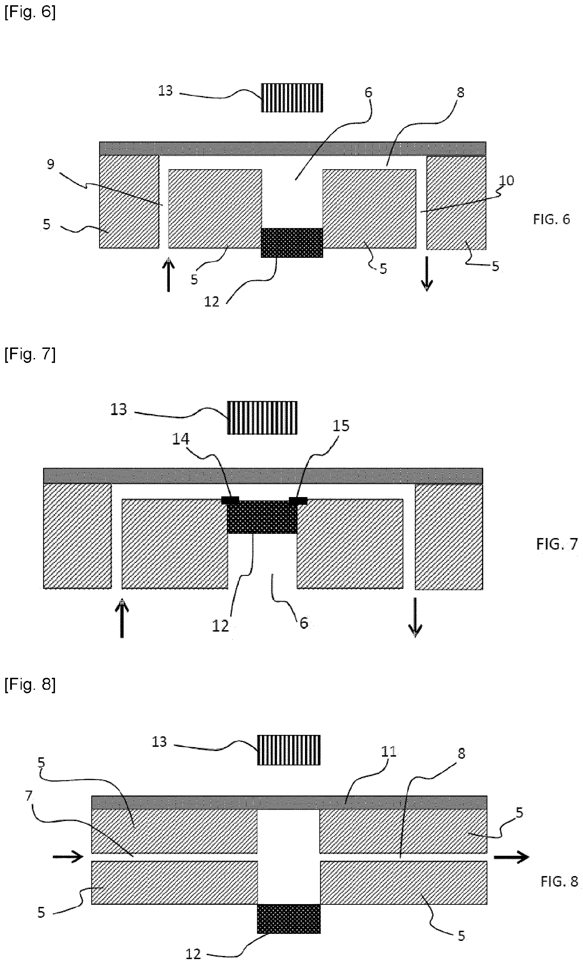

La figure 6 illustre un mode de réalisation dans lequel le transducteur 12 est inséré dans la cavité résonante 6. Une telle réalisation permet de définir différents volumes en fonction de l'application visée. Le volume utile de la cavité résonante est alors variable. Les butées 14 et 15 peuvent être disposées à différents endroits sur la paroi interne de la cavité résonante de façon à positionner la tête du transducteur à des positions prédéterminées. Sur la figure 7, ces butées sont disposées au sommet de la cavité résonante.

Sur la figure 8 est illustré un mode de réalisation dans lequel le canal de passage 7, 8 est réalisé complètement dans l'épaisseur du bloc 5 et débouche dans la cavité résonante à un niveau intermédiaire et non à son sommet. Ce mode de réalisation est bien évidemment compatible avec différentes configurations présentées ci-dessus, c'est-à-dire par exemple avec le transducteur à l'intérieur ou à l'extérieur de la cavité. Mais dans le cas du transducteur à l'intérieur, celui-ci doit rester en dessous de l'arrivée du canal 7 dans la cavité résonante.

En plus de la culture cellulaire, on prévoit avantageusement la fabrication des organoïdes ou sphéroïdes structurés spatialement avec différentes couches de cellules.

La puce microfluidique selon l'invention permet notamment d'injecter des particules ou des cellules dans la cavité résonante, et donc de réaliser des agrégats de cellules dans la cavité. Cela permet de réaliser des cultures cellulaires sur des temps longs en apportant le milieu de culture nécessaire aux cellules agrégées et en lévitation acoustique.

L'invention permet également la création de couches de cellules composites et structurées, ce qui peut être utile dans la perspective de l'ingénierie tissulaire. Pour ce faire, on utilise l'émetteur 13 pour illuminer les cellules à des longueurs d'onde spécifiques et réaliser la technique de l'exclusion optique.

On peut injecter un mélange de deux types de particules ou cellules, celles-ci vont venir former un agrégat mélangeant les deux espèces sous l'action d'ondes acoustiques.

Si on veut organiser spatialement l'agrégat, en particulier structurer l'agrégat en couches successives, annulaires et concentriques, il est possible d'utiliser le principe d'exclusion optique.

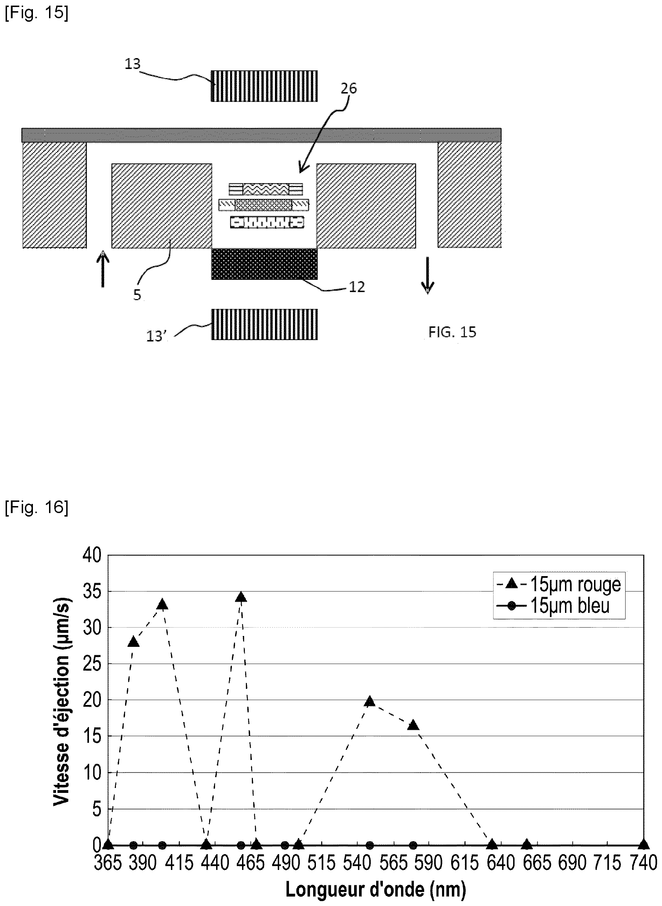

Le principe d'exclusion optique permet d'expulser des particules ou cellules en lévitation acoustique sous l'effet d'une illumination optique à une longueur d'onde donnée adaptée à la cellule ou la particule que l'on souhaite exclure. Cet effet dépend des propriétés d'absorption optique des particules / cellules. Des cellules marquées par un marqueur fluorescent réagissent également à une illumination à la longueur d'onde d'absorption du marqueur fluorescent, voir notamment les Figures 16 à 20.

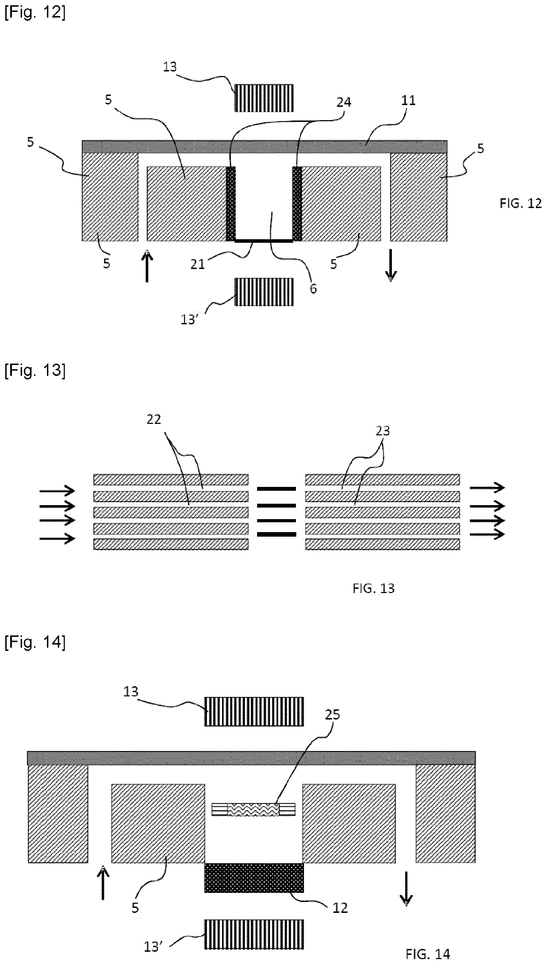

L'invention permet de former un agrégat 2D structuré dans le plan par bandes successives à la périphérie de l'agrégat. On obtient ainsi un agrégat structuré radialement 25 comme illustré sur la Fig. 14. Ceci permet de fabriquer un feuillet composé de bandes circulaires de cellules de natures différentes ce qui peut se révéler utile pour la fabrication des organoïdes complexes.

Pour ce faire, on injecte un mélange de deux cellules C1 et C2, l'une absorbant une longueur d'onde optique donnée $\lambda_{opt1}$ et l'autre ne l'absorbant pas. Dans ce cas, on peut aisément structurer un agrégat. En effet si la zone d'agrégation est illuminée à la longueur d'onde $\lambda_{opt1}$ cela va empêcher C1 de venir s'agréger sous l'effet de la force acoustique. L'espèce C2 va donc former un premier agrégat. Il suffit ensuite de stopper l'illumination à la longueur d'onde $\lambda_{opt1}$ pour que l'espèce C1 vienne s'agréger autour du premier agrégat.

En utilisant à la fois les propriétés optiques et acoustiques, il est donc possible de structurer spatialement des agrégats de cellules en lévitation acoustique. Cette opération peut être répétée plusieurs fois, par injections successives de différents types de cellules marquées ou non et réagissant à différentes longueurs d'onde optiques.

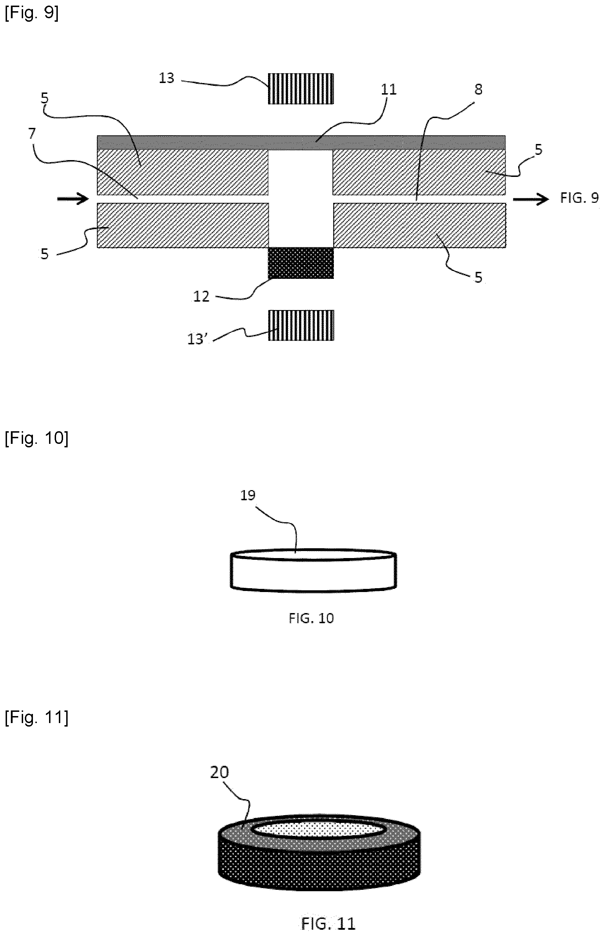

Sur la figure 9, deux émetteurs optiques 13 et 13' sont disposés pour illuminer et/ou d'observer les cellules des deux côtés de la cavité résonante en même temps. Cela permet ainsi d'exclure deux espèces simultanément de la zone d'agrégation acoustique.

Pour ce faire, on prévoit d'utiliser un transducteur piézoélectrique (PZT) 19 transparent comme on le voit sur la figure 10. En effet, il est possible de fabriquer des PZT transparents laissant passer les longueurs d'ondes optiques prévues et donc de s'affranchir de la limitation à une illumination unique d'un seul côté.

On peut également utiliser un transducteur (PZT) annulaire (non packagé). Dans ce cas, il est possible d'illuminer à travers l'anneau et donc de coupler deux sources optiques simultanément.

La double illumination peut également être réalisée avec le mode de réalisation de la figure 12 dans lequel on dispose un transducteur 24 de la forme d'un cylindre creux autour de la cavité résonante. Dans ce cas on prévoit un film 21 faisant l'étanchéité à la base de la cavité résonante et étant transparent pour les faisceaux optiques prévus. Dans ce cas, les deux émetteurs peuvent illuminer librement les cellules dans la cavité résonante.

Un autre avantage de la culture en lévitation est qu'il est possible de créer plusieurs noeuds de pression dans une cavité et ainsi former des agrégats de cellules en lévitation les uns au-dessus des autres. Comme montré sur la Fig. 13, on peut créer de nombreux noeuds, une quinzaine par exemple (ou plusieurs dizaines), dans une cavité haute de quelques mm, 6mm par exemple. Pour ce faire, on peut créer plusieurs microcanaux d'entrée 22 faisant face à plusieurs microcanaux de sortie 23. Chaque couple de microcanaux entrée/sortie peut être prévu dans le plan d'un noeud de pression. Mais la disposition de cellules en lévitation sur plusieurs noeuds de pression peut aussi être réalisée avec un seul canal d'entrée comme le canal de passage.

On peut utiliser l'effet d'exclusion optique sur plusieurs agrégats de cellules en lévitation acoustique et donc structurer des objets dans le volume de la cavité résonante.

La zone d'agrégation est donc centrée sur l'axe du transducteur. Il est alors possible de structurer dans le volume plusieurs agrégats superposés, 26 comme représentés sur la figure 15, chaque agrégat pouvant lui-même être composé de plusieurs bandes circulaires (structuration annulaire) de cellules différentes. Cela permet d'envisager la fabrication en lévitation acoustique de sphéroïdes, d'organoïdes ou de tumoroïdes qui peuvent être maintenus et cultivés en lévitation acoustique.

La création de plusieurs monocouches les unes au-dessus des autres est un gain de temps considérable pour la culture cellulaire.

La présente invention propose donc de nouveaux moyens de culture cellulaire apte à remplacer les techniques de cultures cellulaires traditionnelles sur substrat solides. En effet, dans le cas d'une culture cellulaire traditionnelle, les cellules telles que des cellules souches par exemple, vont se multiplier sur le substrat solide, mais aussi se déplacer pour rentrer en contact avec les autres cellules. On considère la culture terminée lorsque les cellules arrivent « à confluence », c'est-à-dire sont en contact les unes avec les autres et forment ainsi une couche de cellules « monocouche » (une seule couche de cellules).

La présente invention concerne la conception d'un bioréacteur opto-acoustique dans lequel les cellules peuvent être mises en culture en lévitation acoustique et les agrégats cellulaires manipulés et structurés par illumination spécifique de façon à former des agrégats structurés.

Il a été démontré par les inventeurs que l'effet opto-acoustofluidique peut être quantifié par une vitesse d'éjection $V_{ej}$ des objets illuminés. Il s'agit de montrer que les objets en lévitation selon l'invention quittent la zone illuminée avec une vitesse qui est notamment fonction de la longueur d'onde du signal d'illumination. Ces objets sont des micro ou nano particules de tailles comprises entre $0.1\mu m$ et $300\ \mu m$ et sensibles aux longueurs d'ondes utilisées.

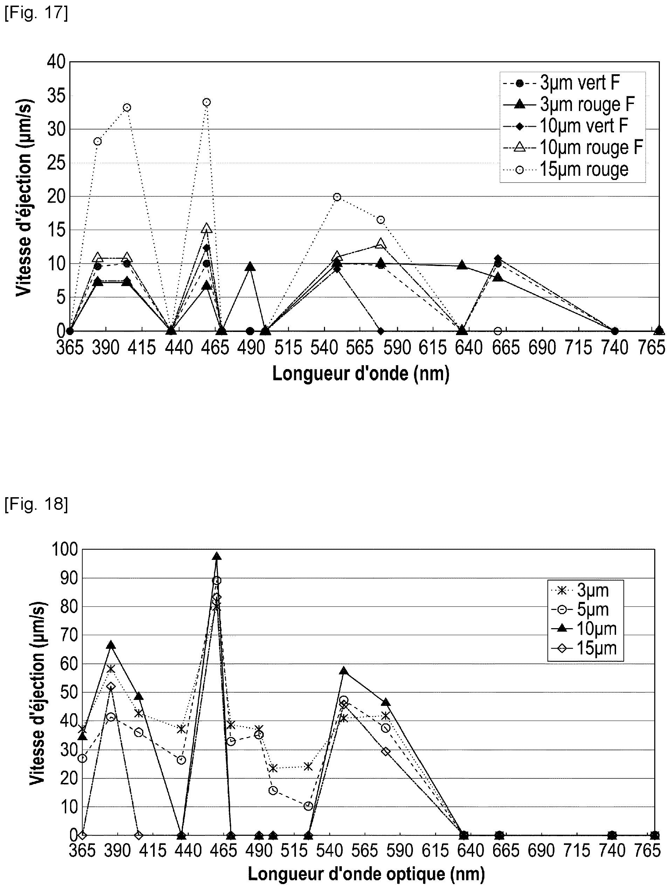

De manière générale, la vitesse d'éjection peut être mesurée pour différentes espèces de particules, en fonction de la longueur d'onde optique, de l'intensité de l'illumination, du grossissement des objectifs du microscope. Ces paramètres permettent de contrôler la puissance de l'illumination. Sur la figure 18 par exemple, on comprend notamment que la taille de la particule joue également un rôle important : plus petite est la particule, plus grande est la vitesse d'éjection.

La figure 16 illustre une vitesse d'éjection d'échantillons ou particules en fonction de la longueur d'onde. Deux groupes de particules ont été formés, l'un avec des particules rouges et l'autre avec des particules bleues de $15\mu m$ de diamètre. Les courbes montrent que la vitesse d'éjection dépend de la longueur d'onde optique mais aussi de la couleur des particules. Les particules rouges sont éjectées de la zone illuminée pour plusieurs longueurs d'onde optique entre 365nm et 770nm, tandis que les particules bleues ne sont jamais éjectées. Dans cet exemple la fréquence acoustique est $f_{ac}$ = 0.650 MHz pour une amplitude A =13 Vpp.

Sur la figure 17, des échantillons suivants ont été analysés dans les mêmes conditions que pour la figure 16 :

- particules vertes fluorescentes de $3\mu m$,
- particules rouges fluorescentes de $3\mu m$,
- particules vertes fluorescentes de $10\mu m$,
- particules rouges fluorescentes de $10\mu m$,
- particules rouges colorées de $15\mu m$.

On constate que la taille des échantillons influence la vitesse d'éjection. On constate également que les particules rouges colorées ont une vitesse d'éjection nettement supérieure à celles des particules fluorescentes.

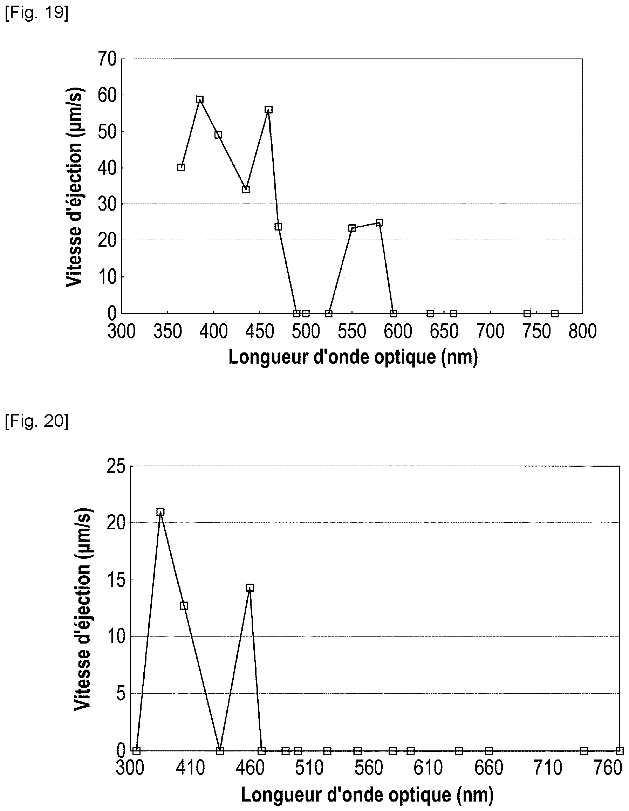

La courbe de la figure 19 concerne la variation de la vitesse d'éjection des globules rouges (GR) (avec une dilution de sang 1: 1000) en fonction de la longueur d'onde. Toutes les expériences ont été effectuées à une fréquence de 751 kHz et une amplitude de 8V, et l'agrégat de sang analysé a été placé au centre de la cavité. On constate que les vitesses d'éjection des GR sont très élevées entre 365nm et environ 490nm, puis entre 515nm et 600nm, au-delà les globules rouges restent agrégés.

La figure 20 concerne une courbe montrant la vitesse d'éjection des globules blancs isolés à partir d'un échantillon de sang total provenant d'un patient sain et marqués au DAPI et CD45 ($\lambda$exc = 415 nm, $\lambda$emission = 500nm), avec des paramètres acoustiques de 751kHz et 8V. La vitesse d'éjection est maximum pour $\lambda_{opt}$ = 385 nm. Les deux autres longueurs d'onde auxquelles les GB marqués réagissent sont $\lambda_{opt}$ = 405 et 460 nm. Les GB ne réagissent pas à d'autres longueurs d'onde.

Le tableau ci-dessous reprend des réponses opto-acoustofluidiques de diverses cellules.

| Cellule | Effet opto-acoustique d'éjection | Paramètres acoustiques | Longueur d'ondes | Applications potentielles |
|---|---|---|---|---|
| Globules rouges | Oui | 746kHz, 8V | 365, 385, 405, 435, 460, 470, 550 et 580 nm | Séparation des cellules sanguines |
| Plaquettes | Oui, dilué (1 :100) | 750kHz, 8V | 385, 405, 435, 460, 550 et 580 nm | Séparation des cellules sanguines |
| Neurones | Non | 745kHz, 8V | toutes | Culture cellulaire, micro cerveau, séparation |
| Cancer du sein (MDA3, MCF-7 et cellules SKBR3) | Non | 745kHz, 8V | toutes | Analyse, Diagnostique, Récupération CTC |
| Cellules Jurkat | Oui, dilué | 740kHz, 8V | 385, 405, 435, 460, 550 et 580 nm | Diagnostic du cancer, cellules CAR-T |
| Sang + cellules cancéreuses | Oui, pour globules rouges | 748kHz, 8V | 365, 385, 405, 435, 460, 470, 550 et 580 nm | Séparation, |
| WBC | oui | 745kHz, 8V | 385, 405, 460 nm | Séparation, récupération pour diagnostic |

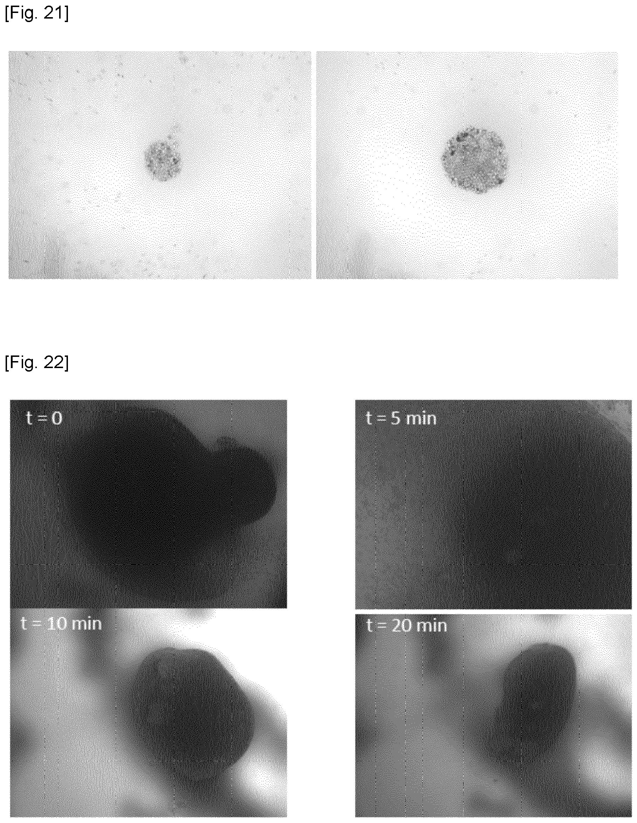

La Fig. 21 montre la formation d'un agrégat de particules blanches à partir d'un mélange de particules de polystyrènes blanches de 10$\mu$m avec des particules rouges de 3$\mu$m. Le mélange est soumis à la force acoustique et illuminé à 460 nm (longueur d'onde à laquelle réagissent les particules rouges), à 60% de la puissance maximale pour un grossissement X10. La fréquence acoustique est de 1.91 MHz avec une amplitude de 9V. La vitesse d'écoulement est de 0.15 ml/h.

On constate la formation d'un agrégat de particules blanches avec très peu de particules rouges piégées. La figure de gauche correspond à un temps de 5 min tandis que la figure de droite montre l'agrégat formé après 20 min. On concentre ainsi progressivement les particules blanches en excluant optiquement les particules rouges.

Les photos de la figure 22 concerne différentes étapes de formation d'agrégat « purifié » en lévitation acoustique par exclusion optique d'un mix de cellules avec écoulement. L'expérience montre l'évacuation des globules rouges par application d'un signal à la longueur d'onde permettant cette évacuation.

Les cellules comprennent des globules rouges (/100) et des cellules cancéreuses MDA (/100). L'illumination est obtenue par un signal à 460 nm, à 80% de la puissance maximale pour un grossissement X10. La fréquence acoustique est de 1.59 MHz avec une amplitude de 10V. La vitesse d'écoulement est de 0.15 ml/h.

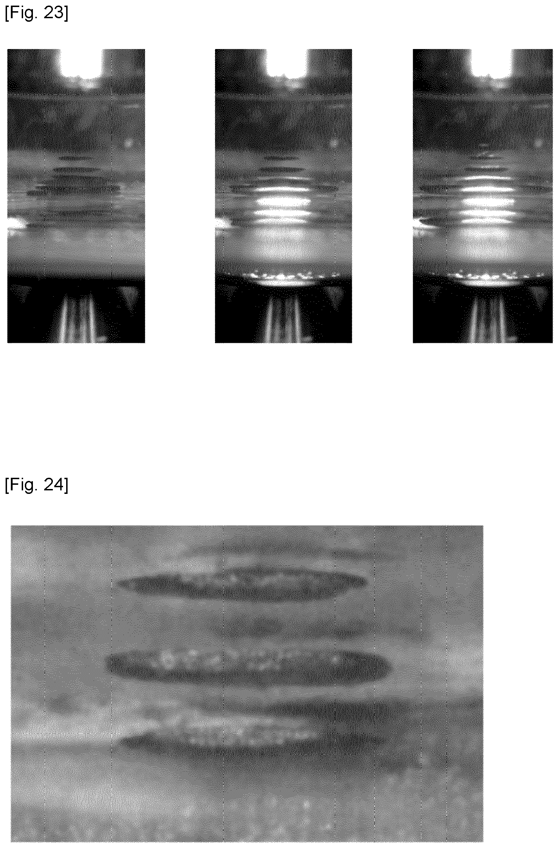

La figure 23 montre l'effet d'exclusion optique appliqué à une cavité multi-noeuds. Sur la photo de gauche on peut voir des agrégats de particules rouges en lévitation acoustique dans une puce microfluidique. Les agrégats forment des couches successives en forme de disques pleins.

Sur la photo du milieu, on applique un éclairage qui atteint d'abord la première couche qui commence par exclure les particules de la zone centrale de façon à constituer une couronne. Partant du bas (proche de l'éclairage), les couches se transforment les unes après les autres en couronne, libérant ainsi à chaque fois le passage de la lumière pour la couche supérieure.

Sur la photo de droite, les couches sont quasiment toutes transformées en couronnes.

Les agrégats sont formés de particules de polystyrène rouge de $15\mu m$. L'illumination est obtenue par un signal à 460 nm, à 60% de la puissance maximale pour un grossissement X10. La fréquence acoustique est de 1.91 MHz avec une amplitude de 9V. Séquence d'illumination : 460 off-on, c'est à dire que l'on commence par former les agrégats en lumière blanche (460 OFF) puis on illumine à 460nm (ON) pour former la couronne de particules en lévitation acoustique.

Sur la figure 24 on distingue une structure annulaire stratifiée. Le mélange de particules comprend des particules de polystyrènes rouges de $15\mu m$ de diamètre et des particules de polystyrènes blanches de $40\mu m$. L'illumination est obtenue par un signal à 460 nm, à 60% de la puissance maximale pour un grossissement X10. La fréquence acoustique est de 1.91 MHz avec une amplitude de 9V. Séquence d'illumination : 460 on-off, c'est à dire que l'on activé simultanément l'acoustique et l'optique, ce qui a exclu les particules rouges du centre. On a ensuite éteint l'illumination à 460 nm, ce qui leur a permis de venir s'agréger sur l'agrégat central.

**Revendications**

1. Puce microfluidique apte à réaliser des manipulations de cellules et/ou leur structuration et/ou leur culture, comprenant :

   - un bloc (5) en matériau biocompatible,
   - un canal de passage (7, 8) réalisé dans le bloc pour le passage de cellules baignant dans un liquide,
   - une cavité résonante (6) réalisée dans le bloc (5), reliée au canal de passage,
   - un générateur (12) d'ondes acoustiques apte à former au moins un agrégat (18, 25) de cellules en lévitation acoustique dans la cavité résonante,
   - au moins un émetteur optique (13) apte à illuminer des cellules dans la cavité résonante à travers au moins une paroi de la cavité résonante et simultanément à la génération d'ondes acoustiques de façon à structurer ledit au moins un agrégat par la technique d'exclusion optique d'une partie des cellules de sorte que seules les cellules insensibles à l'illumination forment un agrégat en une couche,

   la puce **caractérisée en ce que** la cavité résonante comprend des parois pour contenir les cellules provenant du canal de passage, de façon à ce que les cellules ne sont plus sous influence d'un écoulement dans le canal de passage.

2. Puce selon la revendication 1, **caractérisée en ce qu'**elle comprend au moins un deuxième émetteur optique (13'), les deux émetteurs étant disposés de façon à émettre à travers respectivement deux parois opposées de la cavité résonante (6).

3. Puce selon la revendication 1 ou 2, **caractérisée en ce que** les deux émetteurs optiques émettent à des longueurs d'ondes différentes ($\lambda_{opt\,1}$, $\lambda_{opt\,2}$).

4. Puce selon l'une quelconque des revendications précédentes, **caractérisée en ce que** le générateur d'ondes acoustiques comprend un élément supérieur et un élément inférieur prenant en sandwich au moins une partie de la cavité résonante sur deux parois opposées ; l'élément supérieur, fixe ou amovible, étant un transducteur supérieur ou un réflecteur (11) d'ondes acoustiques ; et l'élément inférieur étant un transducteur inférieur (12), les transducteurs étant aptes à émettre les ondes acoustiques ; et **en ce que** l'élément supérieur et/ou l'élément inférieur étant transparent aux faisceaux lumineux prévus pour l'illumination des cellules.

5. Puce selon la revendication 4, **caractérisée en ce que** la cavité résonante (6) est fermée sur son extrémité inférieure par le transducteur inférieur (12).

6. Puce selon la revendication 4, **caractérisée en ce que** le transducteur inférieur (12) est disposé à l'intérieur de la cavité résonante (6).

7. Puce selon la revendication 6, **caractérisée en ce que** la cavité résonante (6) comporte au moins une butée (14, 15) pour bloquer la tête du transducteur inférieur une fois insérée dans la cavité résonante.

8. Puce selon l'une quelconque des revendications 4 à 7, **caractérisée** en ce le transducteur inférieur et/ou le transducteur supérieur est conçu à partir d'un matériau (19) laissant passer les faisceaux optiques prévus pour illuminer les cellules depuis l'extérieur de la cavité.

9. Puce selon l'une quelconque des revendications 4 à 8, **caractérisée** en ce Le transducteur inférieur et/ou le transducteur supérieur a la forme d'un anneau (20) permettant au moins le passage à l'intérieur de l'anneau des faisceaux optiques prévus pour illuminer les cellules depuis l'extérieur de la cavité.

10. Puce selon l'une quelconque des revendications 1 à 4, **caractérisée en ce que** la cavité résonante est fermée sur son extrémité inférieure par un film (16) fixe ou amovible transparent aux ondes acoustiques provenant du transducteur inférieur disposé à l'extérieur de la cavité de résonnance.

11. Puce selon l'une quelconque des revendications 1 à 3, **caractérisée en ce que** le générateur d'ondes acoustiques est un transducteur (24) en forme de cylindre creux disposé autour de la cavité résonante à l'extérieur ou formant parois latérales de la cavité résonante, la paroi supérieure et ou inférieure de la cavité étant en matériau transparent aux faisceaux optiques prévus pour illuminer les cellules depuis l'extérieur de la cavité.

12. Puce selon l'une quelconque des revendications précédentes, **caractérisée en ce que** la cavité résonante (6) est un cylindre dont les parois latérales sont constituées par le bloc.

13. Puce selon la revendication 12, **caractérisée en ce que** le canal de passage débouche dans la cavité résonante à l'extrémité supérieure d'une paroi latérale du cylindre.

14. Puce selon la revendication 13, **caractérisée en ce que** la cavité résonante comporte une butée disposée de façon à ce que la tête du transducteur inférieur (12) puisse être insérée jusqu'à réduire la hauteur du volume utile dans la cavité résonante égale à la hauteur du canal de passage.

15. Puce selon l'une quelconque des revendications précédentes, **caractérisée en ce que** le canal de passage (7, 8) est réalisé sur la surface supérieure du bloc, une lame (11) collée ou amovible couvrant l'ensemble de la surface du bloc y compris l'extrémité supérieure de la cavité résonante ; cette lame étant transparente aux faisceaux optiques prévus pour illuminer depuis l'extérieur les cellules de la cavité résonante ; et **en ce que** lorsqu'un transducteur est disposé en face, cette lame sert de réflecteur réfléchissant sur le côté interne de la cavité résonante les ondes acoustiques du transducteur.

16. Puce selon la revendication 15, **caractérisée en ce que** le canal de passage (7, 8) et le cylindre sont disposés perpendiculairement l'un par rapport à l'autre, et **en ce que** le bloc comprend en outre deux microcanaux (9, 10) traversant le bloc de part en part, parallèlement au cylindre (6) et connectés respectivement aux deux extrémités libres du canal de passage (7, 8) ; le premier microcanal (9) étant destiné à l'arrivée de cellules dans le canal de passage et le deuxième microcanal (10) étant destiné à l'évacuation de cellules depuis le canal de passage.

17. Puce selon l'une quelconque des revendications 4 à 16, **caractérisée en ce que** le réflecteur (11) est une lame en verre, en Polyméthacrylate de méthyle (PMMA), en quartz, en silicium, en polydiméthylsiloxane (PDMS) ou en copolymère oléfinique cyclique (COC).

18. Puce selon l'une quelconque des revendications 4 à 17, **caractérisée en ce que** le réflecteur (11) est conçu à partir d'un matériau identique à celui du bloc et présente une surface interne traitée pour la réflexion d'ondes acoustiques.

19. Puce selon l'une quelconque des revendications précédentes, **caractérisée en ce qu'**elle comprend plusieurs microcanaux (22, 23) réalisés dans l'épaisseur du bloc et débouchant dans la cavité résonante pour des entrées et/ou des sorties de cellules.

20. Puce selon l'une quelconque des revendications précédentes, **caractérisée en ce que** la hauteur de la cavité résonante est fonction du nombre de noeuds de pression à créer et de la longueur d'onde des ondes acoustiques générées par le générateur.

21. Puce selon l'une quelconque des revendications précédentes, **caractérisée en ce que** le bloc (5) est en polydiméthylsiloxane (PDMS) ou en copolymère oléfinique cyclique (COC).

**22.** Puce selon l'une quelconque des revendications précédentes, **caractérisée en ce que** la cavité résonante est dimensionnée avec une hauteur supérieure au diamètre du canal de passage débouchant dans cette cavité résonante.

**23.** Puce selon l'une quelconque des revendications précédentes, **caractérisée en ce que** la cavité résonante (6) présente un diamètre entre 1 et 50mm, la hauteur de la cavité résonante étant comprise entre 5 et 15mm et la hauteur du canal de passage étant égale à 450µm.

**24.** Puce selon l'une quelconque des revendications précédentes, **caractérisée en ce qu'**elle comprend en outre au moins un microcanal supplémentaire réalisé dans le bloc sur le même plan que le canal de passage.

**25.** Procédé de manipulation de cellules en lévitation acoustique dans une puce microfluidique selon l'une quelconque des revendications précédentes, ce procédé comprenant les étapes suivantes :

- injection de cellules dans la cavité résonante (6) via une entrée du canal de passage (7, 8),
- génération d'ondes acoustiques pour mettre en lévitation acoustique les cellules injectées de façon à former un agrégat (18) de cellules en au moins une couche,
- au moins une phase d'illumination des cellules simultanément au maintien des ondes acoustiques de façon à manipuler des cellules selon le principe d'exclusion optique.

**26.** Procédé selon la revendication 25, dans lequel procédé les cellules injectées sont de natures différentes, l'étape de génération d'ondes acoustiques et d'illumination étant réalisées de la manière suivante :

- génération d'ondes acoustiques pour mettre en lévitation acoustique des cellules injectées et en même temps application d'un faisceau lumineux à une longueur d'onde permettant le principe d'exclusion d'une partie des cellules de sorte que seules les cellules insensibles à cette longueur d'onde optique forment un agrégat en une couche,
- maintien des ondes acoustiques et arrêt du faisceau lumineux de sorte que les cellules sensibles à la longueur d'onde du faisceau lumineux viennent maintenant s'agréger en périphérie de l'agrégat déjà formé, et obtenir ainsi un agrégat structuré radialement.

**27.** Procédé selon la revendication 25 ou 26 pour la réalisation d'une structure tridimensionnelle formée de plusieurs couches d'agrégats :

- l'étape d'injection comprenant l'injection de cellules dans la cavité résonante via une ou plusieurs entrées, et
- l'étape de génération d'ondes acoustiques comprenant en outre la génération d'ondes acoustiques pour mettre en lévitation acoustique plusieurs agrégats de cellules injectées sur plusieurs niveaux, les niveaux étant des noeuds de pression acoustique dont le nombre est fonction de la longueur d'onde des ondes acoustiques et de la hauteur de la cavité résonante.

**28.** Procédé selon l'une quelconque des revendications 25 à 27, **caractérisée en ce qu'**il comprend en outre une étape de réalisation d'une culture cellulaire en maintenant l'agrégat ou les agrégats obtenus immobiles en lévitation acoustique pendant la durée de la culture.

**Patentansprüche**

**1.** Mikrofluidischer Chip, der geeignet ist, Zellmanipulationen und/oder ihre Strukturierung und/oder Kultur durchzuführen, umfassend:

- einen Block (5) aus biokompatiblem Material,
- einen in dem Block ausgebildeten Durchgangskanal (7, 8) für den Durchgang von in einer Flüssigkeit schwimmenden Zellen,
- einen Resonanzhohlraum (6), der in dem Block (5) hergestellt und mit dem Durchgangskanal verbunden ist,
- einen Generator (12) zur Erzeugung von Schallwellen, der geeignet ist, mindestens ein Aggregat (18, 25) von akustisch schwebenden Zellen in dem Resonanzhohlraum zu bilden,
- mindestens einen optischen Sender (13), der geeignet ist, Zellen in dem Resonanzhohlraum durch mindestens eine Wand des Resonanzhohlraums zu beleuchten und gleichzeitig Schallwellen zu erzeugen, um das mindestens eine Aggregat durch die Technik des optischen Ausschlusses eines Teils der Zellen zu strukturieren, so dass nur die für die Beleuchtung unempfindlichen Zellen ein einschichtiges Aggregat bilden,
- den Chip, der **dadurch gekennzeichnet ist, dass** der Resonanzhohlraum Wände umfasst, um die Zellen aus dem Durchgangskanal einzuschließen, so dass die Zellen nicht mehr unter dem Einfluss einer Strömung in dem Durchgangskanal stehen.

2. Chip nach Anspruch 1, **dadurch gekennzeichnet, dass** er mindestens einen zweiten optischen Sender (13') umfasst, wobei die beiden Sender so angeordnet sind, dass sie durch jeweils zwei gegenüberliegende Wände des Resonanzhohlraums (6) hindurch senden.

3. Chip nach Anspruch 1 oder 2, **dadurch gekennzeichnet, dass** die beiden optischen Sender mit unterschiedlichen Wellenlängen senden ($\lambda_{opt1}$, $\lambda_{opt2}$).

4. Chip nach einem der vorhergehenden Ansprüche, **dadurch gekennzeichnet, dass** der Generator zur Erzeugung von Schallwellen ein oberes Element und ein unteres Element umfasst, die zumindest einen Teil des Resonanzhohlraums an zwei gegenüberliegenden Wänden zwischen sich einschließen; wobei das obere Element, das fest oder abnehmbar sein kann, ein oberer Wandler oder ein Reflektor (11) für Schallwellen ist; und das untere Element ein unterer Wandler (12) ist, wobei die Wandler geeignet sind, die Schallwellen abzugeben; und dadurch, dass das obere Element und/oder das untere Element für die Lichtstrahlen, die zur Beleuchtung der Zellen vorgesehen sind, transparent sind.

5. Chip nach Anspruch 4, **dadurch gekennzeichnet, dass** der Resonanzhohlraum (6) an seinem unteren Ende durch den unteren Wandler (12) verschlossen ist.

6. Chip nach Anspruch 4, **dadurch gekennzeichnet, dass** der untere Wandler (12) innerhalb des Resonanzhohlraums (6) angeordnet ist.

7. Chip nach Anspruch 6, **dadurch gekennzeichnet, dass** der Resonanzhohlraum (6) mindestens einen Anschlag (14, 15) aufweist, um den Kopf des unteren Wandlers zu blockieren, nachdem er in den Resonanzhohlraum eingeführt wurde.

8. Chip nach einem der Ansprüche 4 bis 7, **dadurch gekennzeichnet, dass** der untere Wandler und/oder der obere Wandler aus einem Material (19) gefertigt ist, das die optischen Strahlen, die zur Beleuchtung der Zellen von außerhalb des Hohlraums vorgesehen sind, durchlässt.

9. Chip nach einem der Ansprüche 4 bis 8, **dadurch gekennzeichnet, dass** der untere Wandler und/oder der obere Wandler die Form eines Rings (20) hat, der zumindest den Durchgang von optischen Strahlen, die zur Beleuchtung der Zellen von außerhalb des Hohlraums vorgesehen sind, in das Innere des Rings ermöglicht.

10. Chip nach einem der Ansprüche 1 bis 4, **dadurch gekennzeichnet, dass** der Resonanzhohlraum an seinem unteren Ende durch eine feste oder entfernbare Folie (16) verschlossen ist, die für Schallwellen durchlässig ist, die von dem unteren Wandler ausgehen, der außerhalb des Resonanzhohlraums angeordnet ist.

11. Chip nach einem der Ansprüche 1 bis 3, **dadurch gekennzeichnet, dass** der Generator zur Erzeugung von Schallwellen ein hohlzylinderförmiger Wandler (24) ist, der um den Resonanzhohlraum herum außerhalb des Resonanzhohlraums angeordnet ist oder Seitenwände des Resonanzhohlraums bildet, wobei die obere und/oder untere Wand des Hohlraums aus einem Material besteht, das für die optischen Strahlen, die zum Beleuchten der Zellen von außerhalb des Hohlraums vorgesehen sind, durchlässig ist.

12. Chip nach einem der vorhergehenden Ansprüche, **dadurch gekennzeichnet, dass** der Resonanzhohlraum (6) ein Zylinder ist, dessen Seitenwände durch den Block gebildet werden.

13. Chip nach Anspruch 12, **dadurch gekennzeichnet, dass** der Durchgangskanal am oberen Ende einer Seitenwand des Zylinders in den Resonanzhohlraum mündet.

14. Chip nach Anspruch 13, **dadurch gekennzeichnet, dass** der Resonanzhohlraum einen Anschlag aufweist, der so angeordnet ist, dass der Kopf des unteren Wandlers (12) eingeführt werden kann, bis die Höhe des Nutzvolumens im Resonanzhohlraum auf die Höhe des Durchgangskanals reduziert ist.

15. Chip nach einem der vorhergehenden Ansprüche, **dadurch gekennzeichnet, dass** der Durchgangskanal (7, 8) auf der oberen Fläche des Blocks ausgeführt ist, wobei eine aufgeklebte oder abnehmbare Lamelle (11) die gesamte Fläche des Blocks einschließlich des oberen Endes des Resonanzhohlraums bedeckt; wobei diese Lamelle für die Lichtstrahlen durchlässig ist, die vorgesehen sind, um die Zellen des Resonanzhohlraums von außen zu beleuchten; und dadurch dass, wenn ein Wandler gegenüber angeordnet ist, diese Lamelle als Reflektor dient, der die Schallwellen des Wandlers auf die Innenseite des Resonanzhohlraums reflektiert.

16. Chip nach Anspruch 15, **dadurch gekennzeichnet, dass** der Durchgangskanal (7, 8) und der Zylinder senkrecht zueinander angeordnet sind und dass der Block außerdem zwei Mikrokanäle (9, 10) umfasst, die den Block durch und durch parallel zum Zylinder (6) durchqueren und jeweils mit den beiden freien Enden des Durchgangskanals (7, 8) verbunden sind; wobei der erste Mikrokanal (9) dazu bestimmt ist, Zellen in den Durchgangskanal einzuführen, und der zweite Mikrokanal (10) dazu bestimmt ist, Zellen aus dem Durchgangskanal abzuführen.

17. Chip nach einem der Ansprüche 4 bis 16, **dadurch gekennzeichnet, dass** der Reflektor (11) eine Lamelle aus Glas, Polymethylmethacrylat (PMMA), Siliziumquarz, Polydimethylsiloxan (PDMS) oder einem Cyclo-Olefin-Copolymer (COC) ist.

18. Chip nach einem der Ansprüche 4 bis 17, **dadurch gekennzeichnet, dass** der Reflektor (11) aus demselben Material wie der Block besteht und eine Innenfläche aufweist, die für die Reflexion von Schallwellen behandelt wurde.

19. Chip nach einem der vorhergehenden Ansprüche, **dadurch gekennzeichnet, dass** er mehrere Mikrokanäle (22, 23) umfasst, die in der Dicke des Blocks ausgeführt sind und in den Resonanzhohlraum münden, um Zelleingänge und/oder -ausgänge zu ermöglichen.

20. Chip nach einem der vorhergehenden Ansprüche, **dadurch gekennzeichnet, dass** die Höhe des Resonanzhohlraums von der Anzahl der zu erzeugenden Druckknoten und der Wellenlänge der vom Generator erzeugten Schallwellen abhängt.

21. Chip nach einem der vorhergehenden Ansprüche, **dadurch gekennzeichnet, dass** der Block (5) aus Polydimethylsiloxan (PDMS) oder einem Cyclo-Olefin-Copolymer (COC) besteht.

22. Chip nach einem der vorhergehenden Ansprüche, **dadurch gekennzeichnet, dass** der Resonanzhohlraum mit einer Höhe bemessen ist, die größer ist als der Durchmesser des in diesen Resonanzhohlraum mündenden Durchgangskanals.

23. Chip nach einem der vorhergehenden Ansprüche, **dadurch gekennzeichnet, dass** der Resonanzhohlraum (6) einen Durchmesser zwischen 1 und 50 mm aufweist, wobei die Höhe des Resonanzhohlraums zwischen 5 und 15 mm liegt und die Höhe des Durchgangskanals 450 $\mu$m beträgt.

24. Chip nach einem der vorhergehenden Ansprüche, **dadurch gekennzeichnet, dass** er außerdem mindestens einen zusätzlichen Mikrokanal umfasst, der in dem Block auf derselben Ebene wie der Durchgangskanal ausgebildet ist.

25. Verfahren zur Manipulation von akustisch schwebenden Zellen in einem mikrofluidischen Chip nach einem der vorhergehenden Ansprüche, wobei das Verfahren die folgenden Schritte umfasst:

   - Injektion von Zellen in den Resonanzhohlraum (6) über einen Eingang des Durchgangskanals (7, 8),
   - Erzeugung von Schallwellen, um die injizierten Zellen in ein akustisches Schweben zu versetzen, so dass ein Aggregat (18) von Zellen in mindestens einer Schicht gebildet wird,
   - mindestens eine Phase, in der die Zellen gleichzeitig mit der Aufrechterhaltung der Schallwellen beleuchtet werden, um die Zellen nach dem optischen Ausschlussprinzip zu manipulieren.

26. Verfahren nach Anspruch 25, wobei in dem Verfahren die injizierten Zellen von unterschiedlicher Beschaffenheit sind, wobei der Schritt der Erzeugung von Schallwellen und der Beleuchtung wie folgt durchgeführt werden:

   - Erzeugung von Schallwellen, um injizierte Zellen in ein akustisches Schweben zu versetzen, und gleichzeitig Anwendung eines Lichtstrahls mit einer Wellenlänge, die das Prinzip des Ausschlusses eines Teils der Zellen ermöglicht, so dass nur die Zellen, die für diese optische Wellenlänge unempfindlich sind, ein einschichtiges Aggregat bilden,

- Aufrechterhaltung der Schallwellen und Stoppen des Lichtstrahls, so dass die Zellen, die auf die Wellenlänge des Lichtstrahls reagieren, sich nun am Rand des bereits gebildeten Aggregats anlagern und so ein radial strukturiertes Aggregat bilden.

27. Verfahren nach Anspruch 25 oder 26 zur Herstellung einer dreidimensionalen Struktur, die aus mehreren Schichten von Aggregaten gebildet wird:

- wobei der Injektionsschritt die Injektion von Zellen in den Resonanzhohlraum über einen oder mehrere Eingänge umfasst, und
- wobei der Schritt des Erzeugens von Schallwellen zudem das Erzeugen von Schallwellen umfasst, um mehrere Aggregate von injizierten Zellen auf mehreren Ebenen in ein akustisches Schweben zu versetzen, wobei die Ebenen Schalldruckknoten sind, deren Anzahl von der Wellenlänge der Schallwellen und der Höhe des Resonanzhohlraums abhängt.

28. Verfahren nach einem der Ansprüche 25 bis 27, **dadurch gekennzeichnet, dass** es außerdem einen Schritt zur Durchführung einer Zellkultur umfasst, indem das erhaltene Aggregat oder die erhaltenen Aggregate während der Dauer der Kultur unbeweglich in einem akustischen Schweben gehalten werden.

## Claims

1. Microfluidic chip capable of carrying out manipulations of cells and/or structuring and/or culturing thereof, comprising:

   - a block (5) made from biocompatible material,
   - a passage channel (7, 8) made in the block for the passage of cells bathed in a liquid,
   - a resonant cavity (6) made in the block (5), connected to the passage channel,
   - an acoustic wave generator (12) capable of forming at least one cell aggregate (18, 25) in acoustic levitation in the resonant cavity,
   - at least one optical emitter (13) capable of illuminating cells in the resonant cavity through at least one wall of the resonant cavity and simultaneous with the generation of acoustic waves in such a way as to structure said at least one aggregate by means of the technique of optical exclusion of a portion of the cells such that only the cells that are not sensitive to the illumination form an aggregate in one layer, the chip **characterized in that** the resonant cavity comprises walls for containing the cells originating from the passage channel, in such a way that the cells are no longer under the influence of a flow in the passage channel.

2. Chip according to claim 1, **characterized in that** it comprises at least one second optical emitter (13'), the two emitters being arranged so as to emit through two opposite walls of the resonant cavity (6) respectively.

3. Chip according to claim 1 or 2, **characterized in that** the two optical emitters emit at different wavelengths ($\lambda_{opt\,1}$, $\lambda_{opt\,2}$).

4. Chip according to any one of the preceding claims, **characterized in that** the acoustic wave generator comprises an upper element and a lower element sandwiching at least part of the resonant cavity on two opposite walls; the upper element, which is fixed or removable, being an upper transducer or an acoustic wave reflector (11); and the lower element being a lower transducer (12), the transducers being capable of emitting the acoustic waves; and **in that** the upper element and/or the lower element being transparent to the light beams provided for illuminating the cells.

5. Chip according to claim 4, **characterized in that** the resonant cavity (6) is closed at its lower end by the lower transducer (12).

6. Chip according to claim 4, **characterized in that** the lower transducer (12) is arranged inside the resonant cavity (6).

7. Chip according to claim 6, **characterized in that** the resonant cavity (6) has at least one stop (14, 15) for blocking the head of the lower transducer once it has been inserted in the resonant cavity.

8. Chip according to any one of claims 4 to 7, **characterized in that** the lower transducer and/or the upper transducer are/is designed starting from a material (19) which allows the optical beams provided for illuminating the cells from outside the cavity to pass through.

9.  Chip according to any one of claims 4 to 8, **characterized in that** the lower transducer and/or the upper transducer has the shape of a ring (20) making it possible at least for the optical beams provided for illuminating the cells from outside the cavity to pass into the inside of the ring.

10. Chip according to any one of claims 1 to 4, **characterized in that** the resonant cavity is closed at its lower end by a fixed or removable film (16) which is transparent to the acoustic waves originating from the lower transducer arranged outside the resonant cavity.

11. Chip according to any one of claims 1 to 3, **characterized in that** the acoustic wave generator is a transducer (24) in the shape of a hollow cylinder arranged around the resonant cavity on the outside or forming side walls of the resonant cavity, the upper and/or lower wall of the cavity being made from a material which is transparent to the optical beams provided for illuminating the cells from outside the cavity.

12. Chip according to any one of the preceding claims, **characterized in that** the resonant cavity (6) is a cylinder the side walls of which are constituted by the block.

13. Chip according to claim 12, **characterized in that** the passage channel leads into the resonant cavity at the upper end of a side wall of the cylinder.

14. Chip according to claim 13, **characterized in that** the resonant cavity has a stop arranged so that the head of the lower transducer (12) can be inserted to reduce the height of the usable volume in the resonant cavity until it is equal to the height of the passage channel.

15. Chip according to any one of the preceding claims, **characterized in that** the passage channel (7, 8) is made on the upper surface of the block, a bonded or removable strip (11) covering all of the surface of the block, including the upper end of the resonant cavity; this strip being transparent to the optical beams provided for illuminating the cells of the resonant cavity from the outside; and **in that**, when a transducer is arranged opposite, this strip acts as a reflector reflecting the acoustic waves from the transducer on the internal side of the resonant cavity.

16. Chip according to claim 15, **characterized in that** the passage channel (7, 8) and the cylinder are arranged perpendicular to one another, and **in that** the block moreover comprises two microchannels (9, 10) passing through the block from one side to the other, parallel to the cylinder (6) and connected respectively to the two free ends of the passage channel (7, 8); the first microchannel (9) being intended for the arrival of cells in the passage channel and the second microchannel (10) being intended for the evacuation of cells from the passage channel.

17. Chip according to any one of claims 4 to 16, **characterized in that** the reflector (11) is a strip made from glass, from polymethyl methacrylate (PMMA), from quartz, from silicon, from polydimethylsiloxane (PDMS) or from cyclic olefin copolymer (COC).

18. Chip according to any one of claims 4 to 17, **characterized in that** the reflector (11) is designed starting from a material identical to that of the block and has an internal surface treated to reflect acoustic waves.

19. Chip according to any one of the preceding claims, **characterized in that** it comprises several microchannels (22, 23) made in the thickness of the block and leading into the resonant cavity for cells to enter and/or exit.

20. Chip according to any one of the preceding claims, **characterized in that** the height of the resonant cavity is a function of the number of pressure nodes to be created and the wavelength of the acoustic waves generated by the generator.

21. Chip according to any one of the preceding claims, **characterized in that** the block (5) is made from polydimethylsiloxane (PDMS) or from cyclic olefin copolymer (COC).

22. Chip according to any one of the preceding claims, **characterized in that** the resonant cavity is dimensioned with a height greater than the diameter of the passage channel leading into this resonant cavity.

23. Chip according to any one of the preceding claims, **characterized in that** the resonant cavity (6) has a diameter between 1 and 50 mm, the height of the resonant cavity being comprised between 5 and 15 mm and the height of the passage channel being equal to 450 μm.

**24.** Chip according to any one of the preceding claims, **characterized in that** it moreover comprises at least one additional microchannel made in the block in the same plane as the passage channel.

**25.** Method for manipulating cells in acoustic levitation in a microfluidic chip according to any one of the preceding claims, this method comprising the following steps:

- injecting cells into the resonant cavity (6) via an inlet of the passage channel (7, 8),
- generating acoustic waves for acoustically levitating the injected cells so as to form a cell aggregate (18) in at least one layer,
- at least one phase of illuminating the cells while simultaneously maintaining the acoustic waves so as to manipulate cells according to the optical exclusion principle.

**26.** Method according to claim 25, in which method the injected cells have different natures, the steps of generating acoustic waves and of illuminating being carried out as follows:

- generating acoustic waves for acoustically levitating the injected cells and at the same time applying a light beam at a wavelength making the principle of exclusion of a portion of the cells possible, so that only the cells not sensitive to this optical wavelength form an aggregate in one layer,
- maintaining the acoustic waves and stopping the light beam so that the cells sensitive to the wavelength of the light beam now form aggregates on the periphery of the aggregate already formed, to thus obtain a radially structured aggregate.

**27.** Method according to claim 25 or 26 for producing a three-dimensional structure formed of several layers of aggregates:

- the step of injecting comprising the injection of cells into the resonant cavity via one or more inlets, and
- the step of generating acoustic waves moreover comprising the generation of acoustic waves for acoustically levitating several aggregates of cells injected on several levels, the levels being acoustic pressure nodes the number of which is a function of the wavelength of the acoustic waves and the height of the resonant cavity.

**28.** Method according to any one of claims 25 to 27, **characterized in that** it moreover comprises a step of carrying out a cell culturing while holding the aggregate or the aggregates obtained immobile in acoustic levitation for the duration of the culturing.

[Fig. 1]

1

3

2

4

FIG. 1

ART ANTERIEUR

[Fig. 2]

13

11

7

5

9

8

10

6

FIG. 2

12

[Fig. 3]

FIG. 3

14

9

1 à 30 mm

5 à 30 mm

10

15

20 à 100 mm

[Fig. 4]

FIG. 4

13

7

17

18

6

8

11

9

10

5

5

5

5

12

16

[Fig. 5]

FIG. 5

13

7

6

8

9

10

5

5

5

5

12

[Fig. 6]

13     6     8

9

5

10

FIG. 6

5

12

[Fig. 7]

13

14     15

FIG. 7

12     6

[Fig. 8]

13     11     8

5

7

5

FIG. 8

5

12

[Fig. 9]

FIG. 9

[Fig. 10]

FIG. 10

[Fig. 11]

FIG. 11

[Fig. 12]

FIG. 12

[Fig. 13]

FIG. 13

[Fig. 14]

FIG. 14

[Fig. 15]

FIG. 15

[Fig. 16]

[Fig. 17]

[Fig. 18]

[Fig. 19]

[Fig. 20]

[Fig. 21]

[Fig. 22]

[Fig. 23]

[Fig. 24]

**RÉFÉRENCES CITÉES DANS LA DESCRIPTION**

**Documents brevets cités dans la description**

• WO 2019002551 A1

**Littérature non-brevet citée dans la description**

• **LEKÄMPER et al.** *Lab on a Chip,* vol. 15 (1), 290-300